# EUROPEAN PATENT SPECIFICATION

(11) **EP 2 147 656 B1**
(45) Date of publication and mention of the grant of the patent: **08.08.2012**
(21) Application number: 09007698.5
(22) Date of filing: 10.06.2009
(51) Int. Cl.: A61B 19/00, B08B 3/12, B08B 9/027

(54) **Endoscope washing and disinfecting apparatus and method of washing endoscope using the endoscope washing apparatus**
Reinigungs- und Desinfektionsgerät für Endoskope und Verfahren zum Reinigen von Endoskopen mit dem Reinigungs- und Desinfektionsgerät für Endoskope
Appareil de nettoyage et de désinfection d'endoscope, et procédé de nettoyage d'endoscope utilisant l'appareil de nettoyage d'endoscope

(30) Priority: 24.07.2008 JP 2008191224
(43) Date of publication of application: 27.01.2010
(73) Proprietor: Olympus Medical Systems Corp., Tokyo 151-0072 (JP)
(72) Inventor: Onishi, Hideto, Tokyo 151-0072 (JP); Hasegawa, Hitoshi, Tokyo 151-0072 (JP); Sewake, Ryuta, Tokyo 151-0072 (JP); Tomita, Masahiko, Tokyo 151-0072 (JP); Noguchi, Toshiaki, Tokyo 151-0072 (JP)
(74) Representative: von Hellfeld, Axel

(56) References cited:
- EP-A- 1 815 782
- WO-A-2007/008337
- GB-A- 1 582 060
- US-A- 5 344 494
- US-A- 6 027 572

## Description

### BACKGROUND OF THE INVENTION

### 1. Field of the Invention

The present invention relates to an endoscope washing and disinfecting apparatus which automatically washes and disinfects an endoscope, and a method of washing an endoscope using the endoscope washing and disinfecting apparatus.

### 2. Description of the Related Art

In recent years, endoscopes have been widely used in the medical field and industrial field. With the endoscopes used in the medical field, the elongated insertion portions are inserted into body cavities, whereby organs in body cavities can be observed, and various treatments can be performed with use of treatment instruments inserted into the insertion channels of the treatment instruments included by the endoscopes in accordance with necessity.

The endoscopes of the medical field are used by being inserted into body cavities especially for the purpose of inspection and treatment, and therefore, they need to be washed and disinfected after use to be used again. As the method for washing and disinfecting the used endoscopes, there is a known method, for example, of using the endoscope washing and disinfecting apparatus (hereinafter, simply called a washing and disinfecting apparatus).

With use of the washing and disinfecting apparatus, an endoscope is automatically subjected to washing, disinfection, rinsing, draining and the like (hereinafter, called a washing and disinfecting process) by only being set in a washing and disinfecting bath of the washing and disinfecting apparatus. On this occasion, a washing solution and a disinfectant solution are supplied to not only an outer surface of the endoscope, but also into a plurality of endoscope channels such as a known gas-supply and water-supply channel, suction channel, and a treatment instrument insertion channel which the endoscope has therein, and thereby, the endoscope is washed and disinfected.

Further, in endoscope channels, particularly in a suction channel and a treatment instrument inserting channel, a tissue and the like in a body cavity which are extracted in an inspection or treatment using the endoscope move along, and therefore, contaminants which are difficult to remove only by the washing process in a washing and disinfecting apparatus easily adhere to the inside of the channels.

This is because in the washing process in the washing and disinfecting apparatus, a step of supplying only a washing solution into the endoscope channels is adopted, but the flow rate in the vicinity of a channel wall of the washing solution which passes inside the channel becomes lower than a flow rate of the washing solution which passes through a center of the channel due to shearing force with the channel wall, and therefore, a contaminant (hereinafter, called a biofilm) which is formed by growth of bacteria firmly adhering to the channel wall is difficult to remove.

Thus, in order to enhance washing performance of the endoscope channel, a user generally washes endoscope channels preliminarily and removes a contaminant adhering to each of the channels by inserting a washing brush with a brush fixed to a distal end of an elongated wire, for example, into the endoscope channel to scrub the endoscope channel prior to washing and disinfection using a washing and disinfecting apparatus.

However, there are problems that it is not only very troublesome for a user, but also increases operation time for washing and disinfecting an endoscope to insert a washing brush into an endoscope channel and perform preliminary washing by scrub. Further, there is a problem that the washing degree differs depending on those who perform preliminary washing.

In view of such problems, there is disclosed a washing and disinfecting apparatus which can reliably scrub, wash and disinfect the inside of an endoscope channel easily in a short time by automatically inserting a washing brush into the endoscope channel, driving the inserted washing brush to advance and retreat, and supplying a washing solution, in, for example, Japanese Patent Application Laid-Open Publication No. 2002-209847. Further, by using such a washing and disinfecting apparatus, scrub is also performed by the washing and disinfecting apparatus, and therefore, the washing degree can be prevented from differing depending on those who perform preliminary washing.

However, when an insertion mechanism of a washing brush to an endoscope channel is provided in the washing and disinfecting apparatus, there are problems of increasing a production cost by increasing the number of components in addition to complicating the configuration of the washing and disinfecting apparatus.

Here, there is a known art which can wash the inside of a channel by supplying gas-liquid two-phase flow in which liquid droplets each with a diameter of, for example, 200 µm formed from a liquid such as water are mixed at a fixed ratio, for example, at a ratio of the droplet to gas of 1 to 1000, into the channel, and causes the liquid droplets at the substantially same flow rate as the gas to collide with a contaminant to remove the contaminant, and the art is disclosed in, for example, Japanese Patent Application Laid-Open Publication No. 2002-505603. Further, an art of using the above art for washing endoscope channels is known.

If washing is performed by using a vapor-liquid two-phase flow, the washing effect equivalent to the case of performing scrub by using a washing brush can be obtained. This is because the flow rate of the gas which flows inside a channel is the same flow rate even in the vicinity of the channel wall or in the center of the channel, unlike that of a liquid, and therefore, the droplets can be also caused to collide against the biofilm firmly adhering to the channel wall at the flow rate of the gas.

Incidentally, when the inside of a channel is washed by using gas-liquid two-phase flow, the flow rate of the gas-liquid two-phase flow becomes higher as the size of the droplet gets smaller. Namely, the flow rate of the gas-liquid two-phase flow is substantially equivalent to the flow rate of the flow with only gas.

However, when the size of the liquid droplet is made small, for example, when it is made about 200 µm as described above, the flow rate of the gas-liquid two-phase flow becomes substantially equivalent to the flow rate of the flow with only gas as described above, and the biofilm in the vicinity of a channel wall can be removed. However, for a large contaminant of a thickness of, for example, 0.5 mm or more, such as a tissue removed from a body cavity, which is accumulated and formed in the channel, the collision ratio of the liquid droplet to the large contaminant decreases since the size of the liquid droplet is small. As a result, the large contaminant is removed while the large contaminant is chipped little by little with collision of the liquid droplets, and therefore, there is the problem that it takes much time to remove a large contaminant.

In contrast to this, the art of increasing the size of each of liquid droplets, for example, making the size of each of the liquid droplets 1 mm, mixing the liquid droplets into gas and causing the liquid droplets each of 1 mm to collide with a contaminant is known. In this case, the collision ratio of the liquid droplet to a large contaminant is larger than the case when the liquid droplets are small, and therefore, the effect of being capable of removing a large contaminant in a short time is provided. However, as the liquid droplet becomes large, the flow rate of the gas-liquid two-phase flow, the flow rate especially in the vicinity of the channel wall becomes lower than the flow rate of the gas, and therefore, there is the problem of being incapable of removing the biofilm adhering to the channel wall.

US 6,027,572 discloses an apparatus for cleaning the interior and the exterior walls of an endoscopy tube, the apparatus comprising an air compressor adapted to supply air to a mixing chamber, wherein the air flow to the mixing chamber is controlled by a pressure regulator. Within the mixing chamber the air supplied by the air compressor is mixed with a cleaning solution so as to produce a turbulent mixture of pressurized air and cleaning solution. This mixture, via a line, is passed to an adapter that provides a pressure-tight fitting to a port of the endoscopy tube.

The present invention is made in view of the above described circumstances, and an object of the present invention is to provide an endoscope washing and disinfecting apparatus including a configuration capable of reliably removing a contaminant inside a channel even with use of gas-liquid two-phase flow, and a method of washing an endoscope using the endoscope washing and disinfecting apparatus.

### SUMMARY OF THE INVENTION

The above mentioned problem is solved by an apparatus according to claim 1 and a method according to claim 7.

Briefly, an endoscope washing and disinfecting apparatus of the present invention is an endoscope washing and disinfecting apparatus which automatically washes and disinfects an endoscope, and includes a gas supply section which supplies a gas to an endoscope channel which the endoscope includes, a liquid supply section which supplies a liquid to the endoscope channel, a fluid supply channel which is connectable to a mouthpiece of the endoscope channel included by the endoscope, and supplies at least one of the gas from the gas supply section and the liquid from the liquid supply section to the endoscope channel, and a liquid droplet mixing section which converts the liquid supplied from the liquid supply section into a liquid droplet, mixes the liquid droplet into the gas flowing in the fluid supply channel with a size of the droplets changeable, and thereby supplies gas-liquid two-phase flow, in which the liquid droplet is mixed at a set ratio into the gas, into the endoscope channel through the fluid supply channel.

Further, a method of washing an endoscope using an endoscope washing and disinfecting apparatus of the present invention is a method of washing an endoscope using an endoscope washing and disinfecting apparatus which automatically washes and disinfects an endoscope, and includes a mixing step in which a liquid droplet mixing section provided in an intermediate position of a fluid supply channel, which is connectable to a mouthpiece of an endoscope channel included by the endoscope, and supplies at least one of a gas from a gas supply section and a liquid from a liquid supply section to the endoscope channel, converts the liquid supplied from the liquid supply section into a liquid droplet and mixes the liquid droplet into the gas which is supplied from the gas supply section and flows in the fluid supply channel with the size of the liquid droplet changeable, and a gas-liquid two-phase flow supply step of supplying a gas-liquid two-phase flow, in which the liquid droplet is mixed at a set ratio into the gas, into the endoscope channel via the fluid supply channel.

The above and other objects, features and advantages of the invention will become more clearly understood from the following description referring to the accompanying drawings.

### BRIEF DESCRIPTION OF THE DRAWINGS

Fig. 1 is a perspective view of a washing and disinfecting apparatus showing a first embodiment;
Fig. 2 is a perspective view of the washing and disinfecting apparatus showing a state in which a top cover of Fig. 1 is opened, and an endoscope can be housed in a washing and disinfecting bath;
Fig. 3 is a diagram showing an internal configuration of the washing and disinfecting apparatus of Fig. 1;
Fig. 4 is a partial cross-sectional view showing an injector of Fig. 3 by enlarging the injector with a channel and a liquid droplet supply channel;
Fig. 5 is a partial cross-sectional view showing a state of removing a large contaminant by causing large liquid droplets mixed in gas-liquid two-phase flow to collide with the large contaminant remaining in an endoscope channel;
Fig. 6 is a partial cross-sectional view schematically showing flow rates of gas-liquid two-phase flow flowing in the endoscope channel in Fig. 5;
Fig. 7 is a partial cross-sectional view showing a state of removing a biofilm by causing liquid droplets smaller than those of Fig. 5, which are mixed in the gas-liquid two-phase flow, to a biofilm remaining in the endoscope channel;
Fig. 8 is a partial cross-sectional view schematically showing flow rates of the gas-liquid two-phase flow flowing in the endoscope channel in Fig. 7;
Fig. 9 is a partial cross-sectional view showing a modified example in which liquid droplets are mixed in gas with use of ultrasound;
Fig. 10 is a diagram schematically showing a configuration of a washing and disinfecting apparatus of a second embodiment which supplies gas-liquid two-phase flow in which ice grains are mixed in gas into an endoscope channel;
Fig. 11 is a partial cross-sectional view schematically showing a state in which the gas-liquid two-phase flow in which ice grains are mixed in the gas is supplied into the endoscope channel, and contaminants are removed;
Fig. 12 is a diagram schematically showing a modified example of a configuration of supplying the gas-liquid two-phase flow with ice grains being mixed in gas into the endoscope channel;
Fig. 13 is a diagram schematically showing a modified example with a configuration which supplies gas-liquid two-phase flow with ice grains being mixed in gas into the endoscope channel, and is different from that of Fig. 12; and
Fig. 14 is a diagram showing a configuration of a modified example of cooling air which cools an electric substrate in a washing and disinfecting apparatus in evaporators in Figs. 12 and 13.

### DETAILED DESCRIPTION OF THE PREFERRED EMBODIMENTS

Hereinafter, embodiments of the present invention will be described with reference to the drawings.

### (First Embodiment)

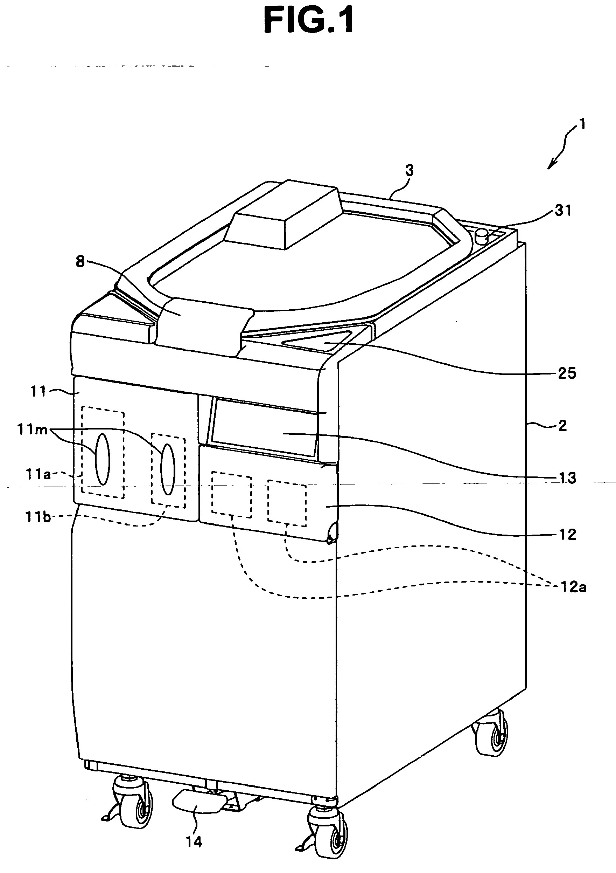

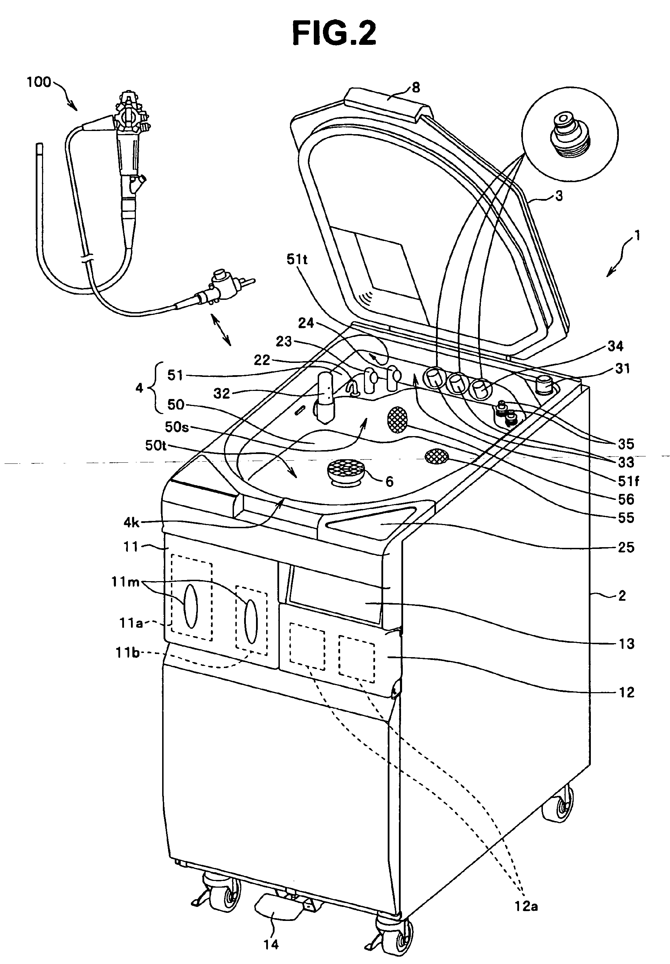

Fig. 1 is a perspective view of a washing and disinfecting apparatus showing the present embodiment, and Fig. 2 is a perspective view of a washing and disinfecting apparatus showing a state in which a top cover of Fig. 1 is opened, and an endoscope can be housed in a washing and disinfecting bath.

As shown in Figs. 1 and 2, a washing and disinfecting apparatus 1 is an apparatus for washing and disinfecting an endoscope 100 which is already used, and a main part is configured by an apparatus main body 2, and a top cover 3 connected to an upper portion of the apparatus main body 2 via, for example, a hinge not illustrated to be openable and closable.

As shown in Fig. 1, in a state in which the top cover 3 is closed to the apparatus main body 2, the apparatus main body 2 and the top cover 3 are configured to be fixed by, for example, a latch 8 which is placed at a position in which the apparatus main body 2 and the top cover 3 are opposed to each other.

A detergent/alcohol tray 11 is placed at an upper portion of a left half part in Fig. 1, for example, which is on a front surface in Fig. 1 to which an operator of the apparatus main body 2 is close (hereinafter, called a front surface) to be able to be drawn forward of the apparatus main body 2.

A detergent tank 11a in which a detergent which is used when the endoscope 100 is washed is stored, and an alcohol tank 11b in which alcohol which is used when the endoscope 100 after washing and disinfection is dried is housed are housed in the detergent/alcohol tray 11. Since the detergent/alcohol tray 11 can be drawn, a predetermined liquid can be supplied to each of the tanks 11a and 11b.

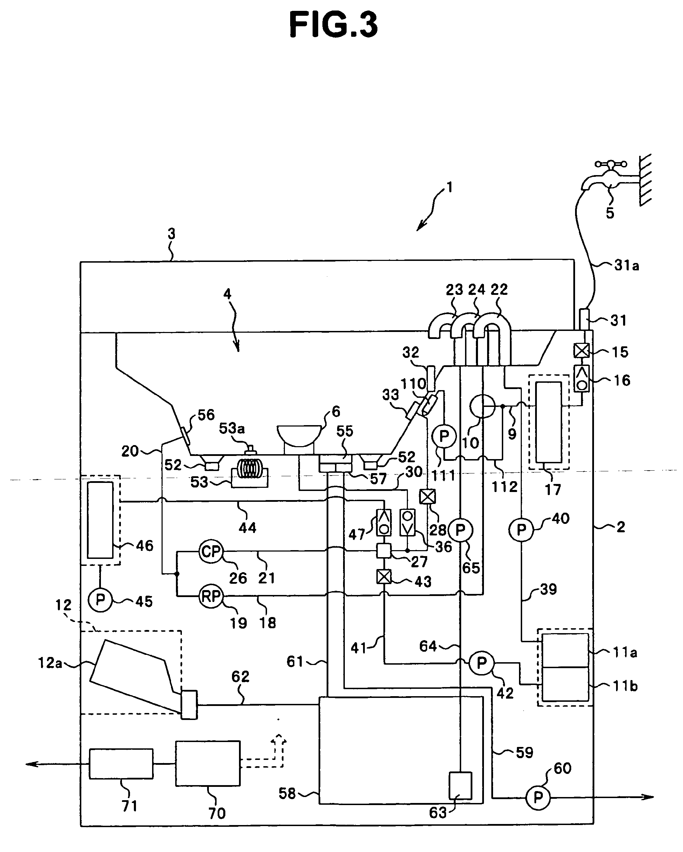

The detergent/alcohol tray 11 is provided with two window portions 11m, so that from the window portions 11m, residual amounts of the detergent and alcohol poured into the respective tanks 11a and 11b can be checked by an operator. The detergent is a concentrated detergent which is diluted to a predetermined concentration by tap water which is subjected to filtering treatment by a supply water filter 17 (see Fig. 3) which will be described later. In the present embodiment, a mixed solution of the detergent and tap water will be called a washing solution in the following description.

Further, at an upper portion of a right half part in Fig. 1, for example, which is on the front surface in Fig. 1 of the apparatus main body 2, a cassette tray 12 is placed to be capable of being drawn forward of the apparatus main body 2. A chemical solution bottle 12a filled with a disinfectant solution such as peracetic acid, for example, which is used when the endoscope 100 is disinfected is housed in the cassette tray 12. Since the cassette tray 12 is capable of being drawn, the chemical solution bottle 12a can be set in a predetermined way.

Further, a sub operation panel 13 on which display of washing and disinfecting time, an instruction button for heating the disinfectant solution, and the like are placed is placed at an upper portion in Fig. 1 of the cassette tray 12, which is on the front surface of the apparatus main body 2.

Further, a pedal switch 14 for opening the top cover 3 which is closed on the upper portion of the apparatus main body 2 upward of the apparatus main body 2 as shown in Fig. 2 by a depressing operation of the operator is placed at a lower portion of the front surface in Fig. 1 of the apparatus main body 2.

Further, as shown in Fig. 2, a main operation panel 25 on which setting switches such as a washing and disinfecting operation start switch of the apparatus main body 2, and a washing and disinfecting mode selection switch are placed is provided near both ends at a front surface side in Fig. 2 where, for example, the operator approaches, of a top surface of the apparatus main body 2.

Further, a water supply hose connection port 31 supplying tap water to the apparatus main body 2 is placed at a back surface side opposed to the front surface which the operator approaches, which is on the top surface of the apparatus main body 2. A water supply hose 31a (see Fig. 3) which will be described later and is connected to a faucet 5 (see Fig. 3) which will be described later is connected to the water supply hose connection port 31. A mesh filter which filters tap water may be placed in the water supply hose connection port 31.

Further, a washing and disinfecting bath 4 capable of housing the endoscope 100, with an endoscope housing port being opened and closed by the top cover 3, is provided at a substantially central portion of the top surface of the apparatus main body 2. The washing and disinfecting bath 4 is configured by a bath main body 50 and a terrace portion 51 continuously provided circumferentially at an outer peripheral edge of the endoscope housing port of the bath main body 50.

The bath main body 50 is capable of housing the endoscope 100 when the endoscope 100 after use is washed and disinfected, and a water drain port 55 for draining a washing solution, water, alcohol, disinfectant solution and the like which are supplied to the bath main body 50 from the bath main body 50 is provided at a bottom surface 50t of the bath main body 50.

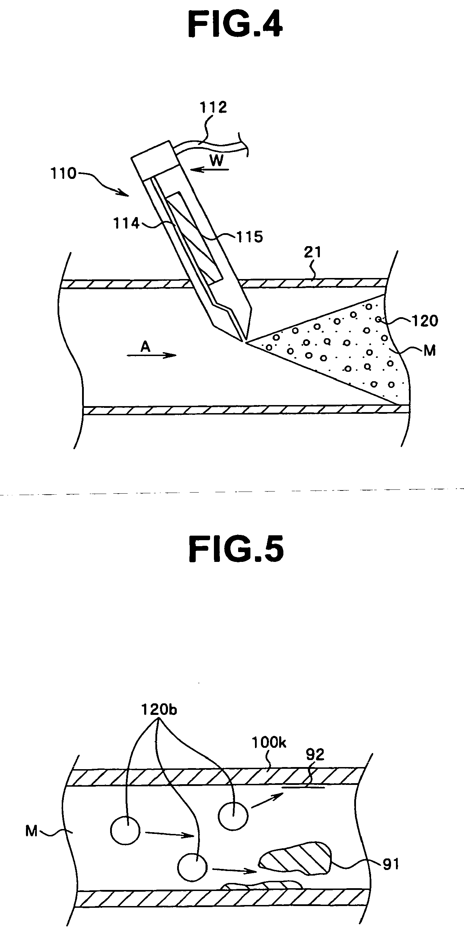

Further, a circulation port 56 for supplying the water, disinfectant solution and the like supplied to the bath main body 50 to an endoscope channel 100k (see Fig. 5) which will be described later included inside the endoscope 100 via means which will be described later is provided at an optional position of a peripheral side surface 50s of the bath main body 50. The circulation port 56 also has a function of supplying the washing solution, water, disinfectant solution and the like supplied to the bath main body 50 into the bath main body 50 again from a water supply circulation nozzle 24 which will be described later through a mesh filter or the like. Further, a mesh filter for filtering the washing solution or the like may be provided in the circulation port 56.

The aforementioned circulation port 56 may be provided at the bottom surface 50t of the bath main body 50. If the circulation port 56 is provided at the bottom surface 50t of the bath main body 50, the timing for supplying various kinds of liquids to the endoscope channel 100k of the endoscope 100, or the bath main body 50 again can be advanced. Further, when the user replaces the mesh filter or the like provided in the circulation port 56, if the circulation port 56 is provided at the bottom surface 50t, it gives an advantage of the operator's easier approach to the circulation port 56.

An ultrasonic transducer 52 which will be described later, a heater 53 (see Fig. 3 for both of them) are also placed in the bath main body 50 of the washing and disinfecting bath 4, and a washing case 6 is placed at a substantially central portion of the bottom surface 50t of the bath main body 50. The ultrasonic transducer 52 gives vibration to the washing water stored in the washing and disinfecting bath 4 or tap water to apply ultrasonic washing to or rinse the outer surface of the endoscope 100. Further, the heater 53 heats the disinfectant solution stored in the washing and disinfecting bath 4, tap water and the like to a predetermined temperature.

The washing case 6 houses buttons such as the scope switches of the endoscope 100, and replaceable components attached to the endoscope 100. Therefore, the respective buttons and the detached components are washed and disinfected together with the endoscope 100.

A water level sensor 32 with a cover which detects water levels of the washing solution, water, disinfectant solution and the like supplied to the bath main body 50 is provided at an optional position of the side surface 50s of the bath main body 50.

On a surface other than the terrace surface 51t of the terrace portion 51, that is, on a surface parallel with the bottom surface 50t of the bath main body 50, a detergent nozzle 22 for supplying the detergent which is diluted to a predetermined concentration by tap water by a detergent pump 40 which will be described later from the detergent tank 11a to the bath main body 50 is placed. Further, on a surface of the terrace portion 51, which is parallel with the bottom surface 50t of the bath main body 50, a disinfectant solution nozzle 23 for supplying the disinfectant solution by a chemical solution pump 65 (see Fig. 3) from a chemical solution tank 58 (see Fig. 3) which will be described later is placed.

Further, on the surface parallel with the bottom surface 50t of the bath main body 50, of the terrace portion 51, the water supply circulation nozzle 24 for supplying the washing solution, water, disinfectant solution and the like for supplying to the bath main body 50, or sucked from the circulation port 56 of the bath main body 50 to the bath main body 50 again is placed.

The detergent nozzle 22, the disinfectant solution nozzle 23 and the water supply circulation nozzle 24 may be placed on the terrace surface 51t.

Further, a plurality of, in this case, two gas supply and water supply/forceps opening ports 33 for supplying gas-liquid two-phase flow, water, alcohol, disinfectant solution, air or the like to a plurality of endoscope channels 100k included in the endoscope 100, a forceps raiser port 34, and a leakage water detecting port 35, which will be described later, are placed on the surface 51fat the side opposed to an operator approaching position 4k, of the terrace surface 51t of the terrace portion 51.

Next, on the basis of Fig. 3, an internal configuration of the washing and disinfecting apparatus 1 will be described. Fig. 3 is a diagram showing the internal configuration of the washing and disinfecting apparatus of Fig. 1.

As shown in Fig. 3, the washing and disinfecting apparatus 1 has the configuration in which the water supply hose connection port 31 is connected to one end of the water supply hose 31a, and the other end of the water supply hose 31a is connected to the faucet 5 which is an external liquid supply section, whereby tap water as a liquid is supplied.

The water supply hose connection port 31 communicates with one end of a water supply channel 9. The water supply channel 9 has the other end connected to a three-way electromagnetic valve 10, and in the middle of the channel, a water supply electromagnetic valve 15, a check-valve 16, and a supply water filter 17 are interposed in sequence from a side of the water supply hose connection port 31.

The supply water filter 17 is configured as a cartridge type filter so as to be regularly replaced, and is attachable and detachable to and from a filter case not illustrated. The supply water filter 17 removes foreign matters, various bacteria and the like in the tap water which passes through the supply water filter 17.

Further, one end of a liquid droplet supply channel 112 is connected to a position of the water supply channel 9 between the three-way electromagnetic valve 10 and the supply water filter 17. The other end of the liquid droplet supply channel 112 is connected to a piezoelectric element injector (hereinafter, simply called an injector) 110 which is a liquid droplet mixing section provided in the intermediate position of a channel 21. Further, a liquid droplet supply pump 111 is interposed in the liquid droplet supply channel 112. The detailed configuration of the injector 110 will be described later.

The three-way electromagnetic valve 10 is connected to one end of a liquid flow channel 18, and switches communication of the water supply channel 9 and the liquid flow channel 18 with the water supply circulation nozzle 24 by an internal valve. Namely, the water supply circulation nozzle 24 communicates with any one of the water supply channel 9 and the liquid flow channel 18 by the switching operation of the three-way electromagnetic valve 10. Further, a liquid flow pump 19 which is a non-self-priming pump capable of transferring only a liquid and excellent in liquid transfer ability is interposed at the other end side of the liquid flow channel 18.

One end of the circulation channel 20 is connected to the circulation port 56 placed in the washing and disinfecting bath 4. The other end of the circulation channel 20 is branched into two so as to communicate with the other end of the liquid flow channel 18 and one end of the channel 21 which is a fluid supply channel. The other end of the channel 21 communicates with the aforementioned respective gas supply and water supply/forceps opening ports 33 (in Fig. 3, only one of the respective gas supply and water supply/forceps opening ports 33 is illustrated). Though not illustrated, the other end of the channel 21 also communicates with the aforementioned forceps raiser port 34.

In the channel 21, a channel pump 26, a channel block 27, and a channel electromagnetic valve 28 are respectively interposed in sequence from the aforementioned one end side, in the middle of the channel. The other end of a case channel 30 with one end connected to a washing case 6 is connected to the channel 21 between the channel block 27 and the channel electromagnetic valve 28. In the case channel 30, a relief valve 36 is interposed.

The channel pump 26 is configured by a self-priming pump capable of transferring either gas or liquid at a higher pressure than a non-self-priming pump. The reason why the channel pump 26 is configured by a self-priming pump is that in order to perform washing, disinfection, rinse and the like reliably for the inside of the endoscope channel 100k included by the endoscope 100, a washing solution, disinfectant solution, tap water, air and the like need to be fed into the endoscope channel 100k from the port 33 via the channel 21 at a high pressure.

In the present embodiment, when the endoscope channel 100k is washed with use of a gas-liquid two-phase flow M which will be described later, it is not necessary to supply the washing solution in the washing and disinfecting bath 4 discharged from the circulation port 56 into the endoscope channel 100k from the channel 21 and the port 33 by driving the channel pump 26. However, when the endoscope channel 100k is washed without using the gas-liquid two-phase flow M, the channel pump 26 is driven, and the washing solution in the washing and disinfecting bath 4 is supplied into the endoscope channel 100k through the channel 21 and the port 33.

Further, the channel pump 26 is driven, the washing solution in the washing and disinfecting bath 4 is supplied to the endoscope channel 100k, and the inside of the endoscope channel 100k is washed, after which, the inside of the endoscope channel 100k may be washed again with use of the air-liquid two-phase flow M. This is because a contaminant may adhere to the inside of the endoscope channel 100k again after washing with only the washing solution.

The detergent nozzle 22 is connected to one end of a detergent channel 39, and the other end of the detergent channel 39 is connected to the detergent tank 11a. In the detergent channel 39, a detergent pump 40 configured by a self-priming pump at a high pressure is interposed in the middle of the detergent channel 39 so as to lift the detergent up to the washing and disinfecting bath 4 from the detergent tank 11a.

The alcohol tank 11b is connected to one end of the alcohol channel 41, and the alcohol channel 41 has the other end connected to the channel block 27 so as to communicate with the channel 21 in a predetermined way.

An alcohol supply pump 42 configured by a self-priming pump at a high pressure for lifting alcohol up to the washing and disinfecting bath 4 from the alcohol tank 11b, and an electromagnetic valve 43 are interposed in the alcohol channel 41.

Further, one end of an air channel 44 is connected to the channel block 27 so as to communicate with the channel 21 in a predetermined way. The air channel 44 is used for supplying only a gas, or air from an air pump 45 configured by a self-priming pump for gas supply for supplying a gas used for the gas-liquid two-phase flow M which will be described later, to the endoscope channel 100k.

The air channel 44 has the other end connected to the air pump 45, and a check-valve 47 and an air filter 46 which is regularly replaced are interposed in the intermediate position of the air channel 44.

A change-over valve 57 capable of being opened and closed for discharging the washing solution or the like to outside and recovering the disinfectant solution to the chemical solution tank 58 by a changing operation of the valve is placed in the water drain port 55 of the washing and disinfecting bath 4.

One end of a water drain channel 59 is connected to the change-over valve 57, and the other end of the water drain channel 59 is connected to and communicates with a water drain hose not illustrated which is connected to an external drain port. A water drain pump 60 configured by a non-self-priming pump is interposed in the water drain channel 59. Further, one end of a chemical solution recovering channel 61 is connected to the change-over valve 57, and the other end of the chemical solution recovering channel 61 is connected to the chemical solution tank 58.

One end of a chemical solution supply channel 62 is connected to the chemical solution tank 58 so as to be supplied with the disinfectant solution from the chemical solution bottle 12a. The other end of the chemical solution supply channel 62 is connected to the cassette tray 12 in a predetermined way.

Further, one end portion with a suction filter 63 being provided, of a chemical solution channel 64 is housed in the chemical solution tank 58 in a predetermined way. The chemical solution channel 64 has the other end connected to the disinfectant solution nozzle 23, and a chemical solution pump 65 configured by a self-priming pump at a high pressure is interposed in the intermediate position in order to lift the disinfectant solution up to the washing and disinfecting bath 4 from the chemical solution tank 58.

For example, the two ultrasonic transducers 52 and the heater 53 are placed beneath the bottom surface 50t of the washing and disinfecting bath 4 as described above. Further, for temperature regulation of the heater 53, a temperature detection sensor 53a is provided in a substantially center of the bottom surface 50t of the washing and disinfecting bath 4.

The heater 53 is for heating the disinfectant solution which is stored in the washing and disinfecting bath 4 and circulates in the apparatus to a predetermined temperature. The disinfectant solution has a proper temperature at which its disinfection effect can be expected the most. The disinfectant solution heated to the aforementioned predetermined temperature that is the proper temperature by the heater 53 can effectively disinfect the endoscope 100 and the respective channels in the apparatus main body 2.

Further, the temperature detection sensor 53a detects the liquid temperature of the disinfectant solution which is stored in the washing and disinfecting bath 4 and circulates in the apparatus, and transmits the detection result to a control unit 70. Subsequently, the control unit 70 performs control of driving and stopping the heater 53 so as to keep the disinfectant solution at a predetermined temperature on the basis of the detection result from the temperature detection sensor 53a.

Further, a power supply 71 which is supplied with electric power from an external AC receptable, and the control unit 70 which is electrically connected to the power supply 71 are provided inside the washing and disinfecting apparatus 1. The control unit 70 performs drive control of the aforementioned respective pumps, respective electromagnetic valves, injector 110 and the like by being supplied with various signals from the main operation panel 25 and the sub operation panel 13.

Next, a configuration of the abovementioned injector 110 will be described by using Fig. 4. Fig. 4 is a partial cross-sectional view showing the injector of Fig. 3 by enlarging it with the channel and the liquid droplet supply channel.

As shown in Figs. 3 and 4, the injector 110 is provided in an intermediate position of the channel 21, more specifically, between the port 33 and the channel electromagnetic valve 28 so that its distal end is inserted into the channel 21. Inside the injector 110, an internal channel 114 which is provided from the distal end of the injector 110 to a proximal end so as to communicate with the liquid droplet supply channel 112, and a piezo element 115 which converts the liquid (hereinafter, called tap water W) flowing in the internal channel 114 into liquid droplets each of a set size by giving vibration are provided.

The injector 110 supplies the gas-liquid two-phase flow M into the endoscope channel 100k included by the endoscope 100 by converting the tap water W supplied from the faucet 5 through the water supply channel 9, and the liquid droplet supply channel 112 into the liquid droplets 120 after the liquid droplet supply pump 111 is driven, and mixing the liquid droplets from the distal end of the internal channel 114 so that the sizes (diameters) of the liquid droplets 120 are changeable at the set ratio to the air A that is the gas flowing in the channel 21, for example, at the ratio of the air A to the tap water W of 1000 to 1.

Change of the size of the liquid droplet 120 in the injector 110 is performed by regulation of the liquid feeding pressure of the tap water W supplied from the faucet 5 after the piezo element 115 is driven to vibrate by drive control of the control unit 70. More specifically, the size of the liquid droplet 120 can be changed to a desired size of the operator.

Specifically, the liquid supply pressure of the liquid droplet supply pump 111 is regulated by drive control of the control unit 70, and thereby, the size of the liquid droplet 120 which is mixed in the air A from the injector 110 is changed. More specifically, when the tap water W is supplied to the injector 110 at a high pressure, the liquid droplet 120 becomes small, whereas when the tap water W is supplied to the injector 110 at a low pressure, the liquid droplet 120 becomes large.

Further, as another method for changing the size of the liquid droplet 120 by the injector 110, there is a method in which after the piezo element 115 is driven by drive control of the control unit 70, a supply voltage which is supplied to the piezo element 115 is changed by voltage control of the control unit 70, and thereby, the size of the liquid droplet 120 which is mixed in the air A from the injector 110 is changed. More specifically, when a high voltage is supplied to the piezo element 115, the vibration of the piezo element 115 becomes large, and therefore, the liquid droplet 120 becomes small. When a low voltage is supplied to the piezo element 115, the vibration of the piezo element 115 becomes small, and therefore, the liquid droplet 120 becomes large.

In the injector 110, by the abovementioned method, the size (diameter) of the liquid droplet 120 can be regulated to about 200 µm in the case of a small liquid droplet, and can be regulated to about 1 mm in the case of a large liquid droplet.

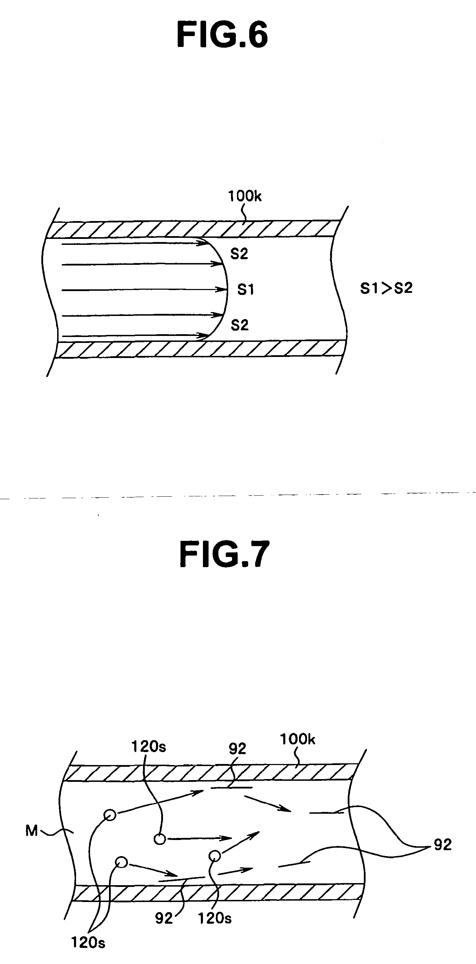

Next, an operation of the present embodiment will be described by using the abovementioned Figs. 3, 4 and 5 to 8. Fig. 5 is a partial cross-sectional view showing a state of removing a large contaminant by causing large liquid droplets mixed in the gas-liquid two-phase flow to collide with a large contaminant remaining in the endoscope channel. Fig. 6 is a partial cross-sectional view schematically showing the flow rate of the gas-liquid two-phase flow flowing in the endoscope channel in Fig. 5.

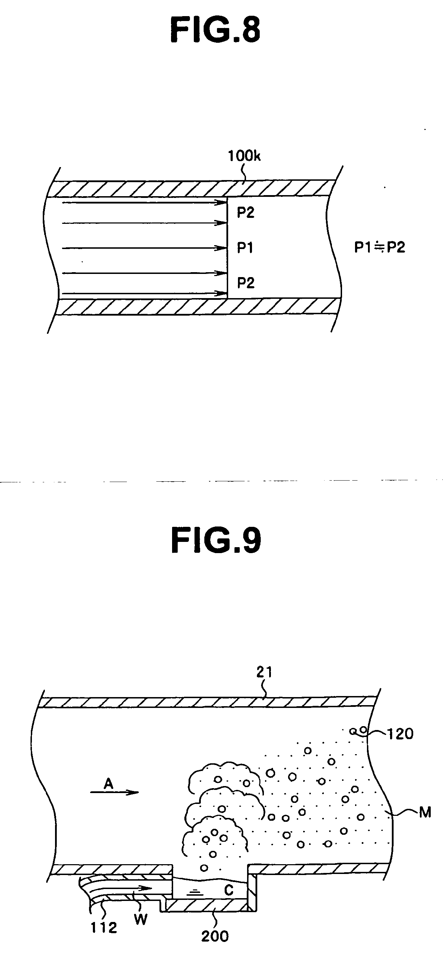

Further, Fig. 7 is a partial cross-sectional view showing the state of removing a biofilm by causing liquid droplets which is mixed in the gas-liquid two-phase flow and are smaller than those in Fig. 5 to collide with the biofilm remaining in the endoscope channel. Fig. 8 is a partial cross-sectional view schematically showing the flow rate of the gas-liquid two-phase flow flowing in the endoscope channel in Fig. 7.

In the operation shown as follows, the operation at the time of washing the endoscope channel 100k of the endoscope 100 which is housed in the washing and disinfecting bath 4 of the washing and disinfecting apparatus 1 will be mainly described. Therefore, the other operations of the washing and disinfecting apparatus 1 than this are known, and therefore, the description thereof will be omitted.

First, when the endoscope 100 housed in the washing and disinfecting bath 4 is washed, each of the gas supply and water supply/forceps opening ports 33 and the forceps raiser port 34 is connected to each of mouthpieces not illustrated of a plurality of endoscope channels 100k included by the endoscope 100 with a connection tube not illustrated.

Thereafter, as shown in Fig. 3, by valve control of the control unit 70, the water supply electromagnetic valve 15 is opened, the three-way electromagnetic valve 10 is switched to the water supply channel 9 side, the air pump 45 is driven by the drive control of the control unit 70, and the channel electromagnetic valve 28 is opened.

As a result, the tap water W which is fed from the faucet 5 through the supply water filter 17 is supplied to the washing and disinfecting bath 4 from the water supply circulation nozzle 24 through the water supply hose 3 1 a, the water supply hose connection port 31, the water supply channel 9, and the channel 21, and the air A which is fed from the air pump 45 through the air filter 46 is supplied into the endoscope channel 100k from each of the ports 33 and 34 through the air channel 44, and the channel 21.

Further, the detergent pump 40 is driven by drive control of the control unit 70, and thereby, the detergent in the detergent tank 11a is supplied to the washing and disinfecting bath 4 from the detergent nozzle 22 through the detergent channel 39. As a result, the outer surface of the endoscope 100 is washed by the washing solution formed by the detergent being diluted with the tap water W.

Thereafter, the liquid droplet supply pump 111 is driven by drive control of the control unit 70, and thereby, the tap water W fed from the faucet 5 is supplied to the injector 110 via the liquid droplet supply channel 112. Thereby, the mixing process of mixing the liquid droplets 120 into a gas flowing in the channel 21 with the size of the liquid droplets 120 changed is performed by the injector 110.

Specifically, in the mixing process, by the drive control of the control unit 70, the liquid droplet supply pump 111 is driven at a low liquid supply pressure, and the tap water W is supplied to the injector 110, whereby, liquid droplets 120b each of a first size which is 1 mm, for example, are mixed into the air A so that the ratio of the air A to the tap water W becomes 1000 to 1. Change of the size of the liquid droplet 120 may be performed by regulation of the voltage which is supplied to the piezo element 115.

Thereafter, a gas-liquid two-phase supply process of supplying the gas-liquid two-phase flow M with the liquid droplets 120b each of the first size mixed in the air A, into the endoscope channel 100k through the ports 33 and 34 is performed. As a result, the large contaminant 91 which remains in the endoscope channel 100k is removed with collision of the liquid droplets 120b as shown in Fig. 5. The large contaminant 91 which is removed and the gas-liquid two-phase flow M are discharged into the washing and disinfecting bath 4.

Further, since at this time, the liquid droplets 120b each of the first size having a size of 1 mm are mixed in the gas-liquid two-phase flow M as shown in Fig. 5, a flow rate S2 in the vicinity of the wall surface is lower than a flow rate S1 in the center of the channel in the endoscope channel 100k as shown in Fig. 6 (S1>S2), a biofilm 92 which firmly adheres to the wall surface of the endoscope channel 100k is difficult to remove as shown in Fig. 5.

After the gas-liquid two-phase flow M is supplied into the endoscope channel 100k for a fixed time in which the large contaminant 91 is removed, the liquid droplet supply pump 111 is driven at a high liquid supply pressure by the drive control of the control unit 70 and the tap water W is supplied to the injector 110 again in the mixing process, whereby the liquid droplets 120s each of a second size of a size of, for example, of 20 µm, which is smaller than the first size are mixed in the air A so that the ratio of the air A to the tap water W becomes 1000 to 1. Change of the size of the liquid droplet 120 may be performed by regulation of the voltage which is supplied to the piezo element 115.

Thereafter, a gas-liquid two-phase supply process in which the gas-liquid two-phase flow M with the liquid droplets 120s each of the second size being mixed in the air A is supplied into the endoscope channel 100k through the ports 33 and 34 is performed. As a result, the biofilm 92 which remains on the wall surface of the endoscope channel 100k is removed with collision of the liquid droplets 120s as shown in Fig. 7.

Since the liquid droplets 120s each of the second size, which have the size of 20 µm and are very small, are only mixed in the gas-liquid two-phase flow M as shown in Fig. 7, the flow rate P1 in the center of the channel and the flow rate P2 in the vicinity of the wall surface are substantially equal (P1 ≈ P2) in the endoscope channel 100k, and the flow rate of the gas-liquid two-phase flow M is substantially the same flow rate as the flow rate of the flow with only the air A, as shown in Fig. 8. From this, as shown in Fig. 7, the liquid droplets 120s are caused to collide against the biofilm 92 firmly adhering to the wall surface of the endoscope channel 100k, and thereby the biofilm 92 can be removed. The biofilm 92 which is removed and the gas-liquid two-phase flow M are discharged into the washing and disinfecting bath 4.

Further, the washing solution in the washing and disinfecting bath 4 and the gas-liquid two-phase flow M which is discharged from the endoscope channel 100k through the circulation port 56 are supplied again from the water supply circulation nozzle 24 to the washing and disinfecting bath 4 through the circulation channel 20 and the liquid flow channel 18 to be used for washing the outer surface of the endoscope 100, as a result that the liquid flow pump 19 is driven and the three-way electromagnetic valve 10 is switched to the liquid flow channel 18 side by the drive control of the control unit 70.

At this time, the channel pump 26 is not driven, and therefore, the washing solution and the gas-liquid two-phase flow M in the washing and disinfecting bath 4 which are discharged from the circulation port 56 are not supplied into the endoscope channel 100k. This is for preventing the washing solution discharged from the circulation port 56 being mixed into the air A flowing in the channel 21 as well as preventing the large contaminant 91 and the biofilm 92 which are discharged into the washing and disinfecting bath 4 with the gas-liquid two-phase flow M from being supplied to the endoscope channel 100k again.

After removal of the biofilm 92 of the endoscope channel 100k, the control unit 70 stops drive of the respective pumps 40, 45, 19 and 111, opens the change-over valve 57, drives the water drain pump 60, and discharges the washing solution and the gas-liquid two-phase flow M from the washing and disinfecting bath 4 through the water drain port 55 and water drain channel 59 from the external drain port.

Thereafter, a rinsing process in which the tap water W is supplied into the washing and disinfecting bath 4, the outer surface of the endoscope 100 is rinsed, and when the channel pump 26 is driven by the control unit 70, the tap water W is supplied to the endoscope channel 100k through the circulation channel 20, the channel 21, and the respective ports 33 and 34 from the circulation port 56 is performed. As a result of the rinsing process, the inside of the endoscope channel 100k washed by the gas-liquid two-phase flow M is rinsed with the tap water W.

Since the subsequent process is known, the description thereof will be omitted hereinafter. Further, prior to the gas-liquid two-phase flow supply process, the channel pump 26 is driven as described above, and the washing solution in the washing and disinfecting bath 4 is supplied into the endoscope channel 100k through the channel 21 and the port 33, whereby the endoscope channel 100k may be washed, prior to washing of the endoscope channel 100k by supply of the gas-liquid two-phase flow M.

Thus, the present embodiment is described as including the configuration in which the injector 110 which mixes the liquid droplets 120 of the tap water W into the air A can change the size of the liquid droplets 120. In other words, it is described that the size of the liquid droplet 120 can changed into a desired size by an operator.

Further, it is described that when the endoscope channel 100k is washed with the gas-liquid two-phase flow M with the liquid droplets 120 mixed therein, the large contaminant 91 in the endoscope channel 100k is removed first with the gas-liquid two-phase M with the liquid droplets 120b each of the first size being mixed, and thereafter, the biofilm 92 in the endoscope channel 100k is removed with the gas-liquid two-phase flow M with the liquid droplets 120s each of the second size which is smaller than the first size being mixed.

According to this, even with use of the gas-liquid two-phase flow M, both the large contaminant 91 and the biofilm 92 remaining in the endoscope channel 100k can be reliably removed in a short time.

Further, since the washing solution does not need to be supplied into the endoscope channel 100k, the use amount of the detergent can be decreased more than conventional amount, and therefore, cost can be reduced.

From above, even with use of the gas-liquid two-phase flow M, the washing and disinfecting apparatus 1 including the configuration capable of removing the contaminant in the endoscope channel 100k and the method of washing the endoscope 100 using the washing and disinfecting apparatus 1 can be provided.

Hereinafter, a modified example will be shown. In the present embodiment, the gas A used for the gas-liquid two-phase flow M is shown with air cited as an example, but it goes without saying that the gas is not limited to this, and the gas may be carbon dioxide or the like.

Further, in the present embodiment, the liquid W used for the gas-liquid two-phase flow M is shown with tap water passing through the supply water filter 17 as an example, but the liquid W is not limited to this, and may be RO water passing through a known RO (Reverse Osmosis) membrane, pure water or the like.

Further, in the present embodiment, the tap water W is mixed into the air A with the ratio of the gas to the liquid droplet of 1000 to 1 in the gas-liquid two-phase flow M, but it goes without saying that the mixture ratio is not limited to this.

Further, the first size of the liquid droplet 120 which is mixed into the gas-liquid two-phase flow M is described as 1 mm, and the second size is described as 20 µm, but it goes without saying that the sizes are not limited to these values.

A modified example will be shown by using Fig. 9 hereinafter. Fig. 9 is a partial cross-sectional view showing a modified example in which a liquid droplet is mixed in gas by using ultrasound.

The present embodiment describes that by the injector 110, the tap water W supplied from the faucet 5 is changed to the liquid droplets 120, the size of the liquid droplets 120 is changed, and the liquid droplets 120 each of a set size are mixed into the air A, whereby the gas-liquid two-phase flow M is supplied to the endoscope channel 100k.

The size of the liquid droplet 120 is changed by other devices than the injector 110, without being limited to the injector 110, and the liquid droplets of the set sizes may be mixed into the air A.

Specifically, as shown in Fig. 9, instead of providing the injector 110, the end portion of the liquid droplet supply channel 112 may be connected to the intermediate position between the port 33 of the channel 21 and the channel electromagnetic valve 28 to communicate with the channel 21, and an ultrasonic transducer 200 which is an ultrasonic generating section configuring a liquid droplet mixing section may be provided in the vicinity of a connection portion.

The ultrasonic transducer 200 has the function of changing the tap water W to the liquid droplets 120 by applying an ultrasound C to the tap water W. Further, the ultrasonic transducer 200 performs control of changing the size of the liquid droplet 120 by the ultrasonic frequency being changed by vibration control of the control unit 70.

By such a configuration, the size of the liquid droplet 120 is changed, and the liquid droplets 120 each of the set size is mixed into the air A flowing in the channel 21, whereby the gas-liquid two-phase flow M can be supplied to the endoscope channel 100k.

### (Second Embodiment)

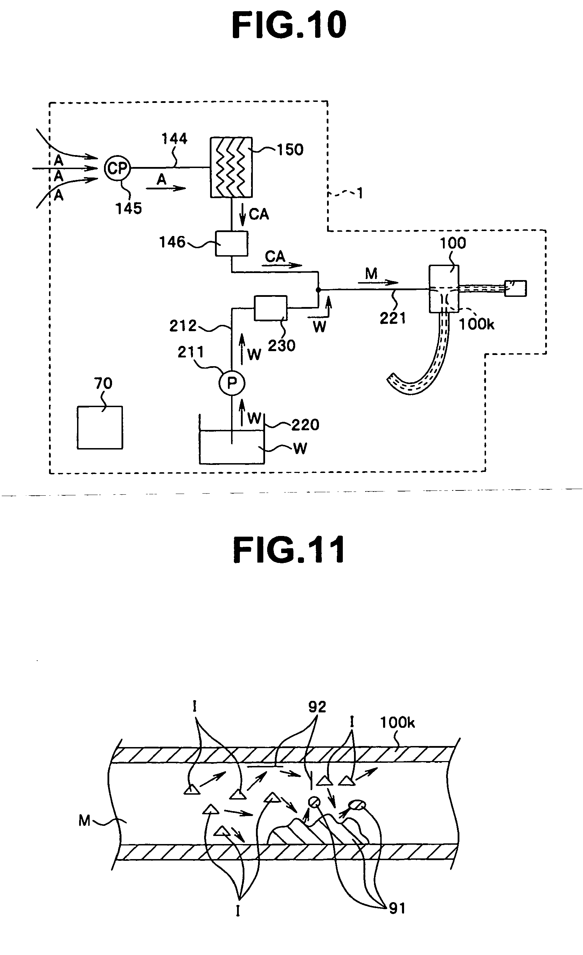

Fig. 10 is a diagram schematically showing a configuration of a washing and disinfecting apparatus of a second embodiment which supplies gas-liquid two-phase flow in which ice grains are mixed in gas is supplied into an endoscope channel, and Fig. 11 is a partial cross-sectional view schematically showing a state in which the gas-liquid two-phase flow in which ice grains are mixed in the gas is supplied into the endoscope channel, and the contaminant is removed.

The configuration of the washing and disinfecting apparatus of the second embodiment differs from the washing and disinfecting apparatus of the first embodiment shown in the abovementioned Figs. 1 to 8 in that the endoscope channel is washed by using the gas-liquid two-phase flow in which ice grains are mixed in the gas instead of liquid droplets. Therefore, only the different point will be described, and the same components as those in the first embodiment are assigned with the same reference numerals and characters, and the description thereof will be omitted.

As shown in Fig. 10, a compressor 145 which is an air supply section which takes in the air A from outside the washing and disinfecting apparatus 1 and supplies the air A into the endoscope channel 100k by drive control of the control unit 70 is provided in the washing and disinfecting apparatus 1 in the present embodiment, and one end of an air supply channel 144 is connected to the compressor 145. The other end of the air supply channel 144 is connected to one end side of a fluid supply channel 221 with the other end side connectable to the mouthpiece of the endoscope channel 100k via a connection tube not illustrated.

A heat exchanger 150 and an air filter 146 are interposed in the air supply channel 144 in sequence from the compressor 145 side. The heat exchanger 150 has the function of cooling the air A supplied from the compressor 145 to make the air A cold air CA.

Further, one end side of a liquid supply channel 212 is connected to a tank 220 which is a liquid supply section in which the cold liquid W is stored so as to communicate with an inside of the tank 220. The other end of the liquid supply channel 212 is connected to one end side of the fluid supply channel 221 together with the other end of the air supply channel 144.

A liquid supply pump 211 and a liquid droplet mixing section 230 are interposed in the liquid supply channel 212 in sequence from the tank 220 side. The liquid supply pump 211 supplies the liquid W in the tank 220 into the endoscope channel 100k by drive control of the control unit 70.

Further, the liquid droplet mixing section 230 converts the droplets mixed in the air A into an ice grain I with, for example, a diameter of 200 µm by converting the liquid W supplied from the tank 220 by drive of the liquid supply pump 211 into liquid droplets, and mixing the liquid droplets into the air A cooled by the heat exchanger 150, and is configured by, for example, an atomizer.

A size of the liquid droplet which is supplied from the liquid droplet mixing section 230 also can be changed by changing the air supply pressure of the liquid supply pump 211. Further, the other components of the washing and disinfecting apparatus 1 are the same as those in the abovementioned first embodiment.

Next, an operation of the present embodiment thus configured will be described by using Figs. 10 and 11. First, when the endoscope channel 100k of the endoscope 100 is washed, the compressor 145 is driven by the control unit 70, whereby the air A outside the washing and disinfecting apparatus 1 is sucked by the compressor 145, and the sucked air A is fed to the air supply channel 144.

Thereafter, the air A is cooled by the heat exchanger 150, and the cooled air CA is supplied to the fluid supply channel 221 through the air filter 146.

Next, when the liquid supply pump 211 is driven by the drive control of the control unit 70, the liquid W in the tank 220 is fed to the liquid supply channel 212, and is converted into liquid droplets by the liquid droplet mixing section 230. Thereafter, the liquid droplets are mixed into the air CA which is cooled by the heat exchanger 150 and flows in the fluid supply channel 221 at the set ratio, for example, at the ratio of the gas to the liquid droplet of 1000 to 1. Thereafter, the liquid droplets are converted into ice grains I with a diameter of, for example, 200 µm by the heat exchanger 150, and the gas-liquid two-phase flow M in which the ice grains I are mixed in the air CA is supplied to the endoscope channel 100k.

As a result, the ice grains I in the gas-liquid two-phase flow M collide with the large contaminant 91 and the biofilm 92 in the endoscope channel 100k as shown in Fig. 11, and thereby, the large contaminant 91 and the biofilm 92 are removed.

The reason why the large contaminant 91 and the biofilm 92 can be removed by the ice grains I is that the ice grains I have a higher collision force to contaminants than the liquid droplets 120 since the ice grains are solid. At this time, large ice grains I may be caused to collide against the large contaminant 91, whereas small ice grains I may be caused to collide against the biofilm 92.

Thus, in the present embodiment, it is described that the gas-liquid two-phase flow M in which the ice grains I are mixed in the air CA at the set ration is supplied into the endoscope channel 100k, and thereby, the endoscope channel 100k is washed.

According to this, the ice grain I has a higher collision force against a contaminant than a liquid droplet, and therefore, can remove the contaminant in a shorter time than in the abovementioned first embodiment. The other effects are the same as those in the abovementioned first embodiment. Further, in the present embodiment, prior to washing of the endoscope channel 100k by supply of the ice grain I, the channel pump 26 may be driven, and the washing solution in the washing and disinfecting bath 4 may be supplied into the endoscope channel 100k through the channel 21 and the port 33, whereby the endoscope channel 100k may be washed.

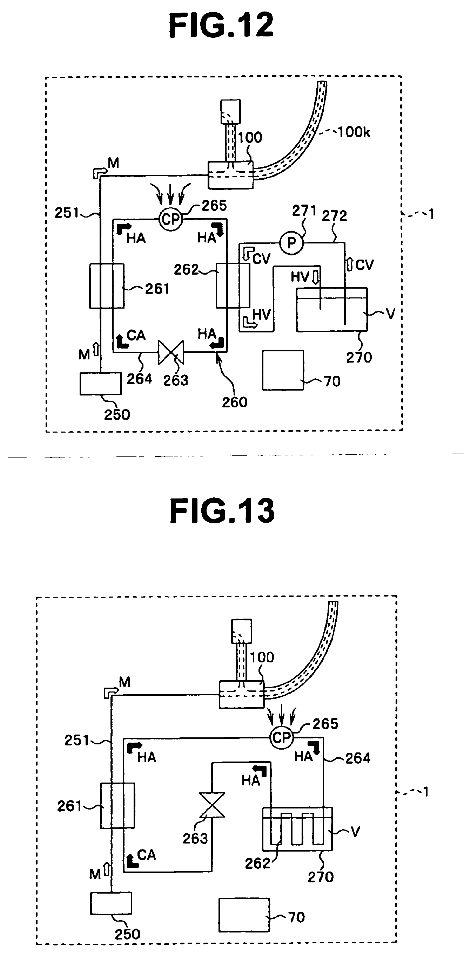

A modified example will be shown hereinafter by using Fig. 12. Fig. 12 is a diagram schematically showing a modified example with the configuration in which gas-liquid two-phase flow in which ice grains are mixed in gas is supplied into the endoscope channel.

As shown in Fig. 12, the washing and disinfecting apparatus 1 may include the configuration which converts the liquid droplets of the gas-liquid two-phase flow M into the ice grains I by a heat pump 260. The main part of the heat pump 260 is configured by a gas circulation channel 264, a heat pump evaporator (hereinafter, simply called an evaporator) 261, a heat pump condenser (hereinafter, simply called a condenser) 262, a compressor 265 provided between the evaporator 261 and the condenser 262 in the gas circulation channel 264, and an expansion valve 263 provided between the evaporator 261 and the condenser 262 in the gas circulation channel 264 at a position different from the position where the compressor 265 is provided. The compressor 265 has the function of circulating air in the gas circulation channel 264.

Specifically, as shown in Fig. 12, a gas-liquid two-phase flow generating unit 250 which supplies the gas-liquid two-phase flow M in which liquid droplets are mixed in the air A at the abovementioned set ratio into the endoscope channel 100k through the fluid supply channel 251 is provided at one end of the fluid supply channel 251 of which the other end is connectable to a mouthpiece not illustrated of the endoscope channel 100k through a connection tube not illustrated, as shown in Fig. 12. As the gas-liquid two-phase flow generating unit 250, the configuration shown in the first embodiment or the like is assumed.

Further, the evaporator 261 is provided in an intermediate position of the fluid supply channel 251. The evaporator 261 has the function of bringing the liquid droplets in the gas-liquid two-phase flow M into a state of ice by being supplied with the air CA which is cooled by the expansion valve 263 after the compressor 265 is driven by drive control of the control unit 70, and also has the function of supplying air HA, which is heated by heat taken from the gas-liquid two-phase flow M, to the condenser 262.

Further, the expansion valve 263 has the function of converting the air HA which is compressed and heated by the condenser 262 into the cold air CA by expending the air HA, and supplying the air CA to the evaporator 261.

The condenser 262 is further interposed in an intermediate position of the liquid circulation channel 272. One end and the other end of a liquid circulation channel 272 communicate with the inside of a tank 270. Further, a liquid circulation pump 271 is interposed in an intermediate position of the liquid circulation channel 272. When the liquid circulation pump 271 is driven by drive control of the control unit 70, a liquid V in the tank 270 circulates in the liquid circulation channel 272 with respect to the tank 270. As the liquid V which is stored in the tank 270, for example, a disinfectant solution or the like is cited. The liquid V is not limited to the disinfectant solution, and may be tap water or the like.

The condenser 262 has the function of compressing the heated air HA which is supplied from the evaporator 261 via the gas circulation channel 264, and heating a cold disinfectant solution CV which is supplied from the tank 270 through the liquid circulation channel 272 by drive of the liquid circulation pump 271 to convert the disinfectant solution CV into a warm disinfectant solution HV and to return the disinfectant solution HV to the tank 270.

The reason why the disinfectant solution is heated is that the disinfectant solution is heated to the abovementioned proper temperature suitable for disinfection and used. Therefore, the disinfectant solution is conventionally heated by the heater 53 (see Fig. 3), but by using the present configuration, heating time by the heater 53 can be decreased.

As above, according to the present configuration, the gas-liquid two-phase flow in which the ice grains I are mixed in the gas also can be supplied to the endoscope channel 100k, and the disinfectant solution V can be heated. The other effects are the same as those of the abovementioned second embodiment.

Hereinafter, another modified example will be shown by using Fig. 13. Fig. 13 is a diagram schematically showing a different modified example from Fig. 12, which has the configuration of supplying the gas-liquid two-phase flow in which ice grains are mixed in the gas into an endoscope channel.

The configuration shown in Fig. 13 differs from the configuration shown in Fig. 12 in that the gas circulation channel and the liquid circulation channel are integrated. Therefore, the same components as in Fig. 12 are assigned with the same reference numerals and characters, and description thereof will be omitted.

As shown in Fig. 13, the evaporator 261 is provided in an intermediate position of the gas circulation channel 264. The compressor 265 is interposed between the evaporator 261 of the gas circulation channel 264 and the tank 270, and the expansion valve 263 is interposed in a position between the evaporator 261 of the gas circulation channel 264 and the tank 270, which is different from the position where the compressor 265 is interposed.

Further, the condenser 262 is provided in a part of the gas circulation channel 264, which is located in the tank 270, and, for example, the disinfectant solution V is stored in the tank 270.

According to such a configuration, when the compressor 265 is driven by drive control of the control unit 70, the air A circulates inside the gas circulation channel 264.

At this time, the air HA which is heated by heat taken from the gas-liquid two-phase flow M when the liquid droplets of the gas-liquid two-phase flow M are brought into a state of ice by the evaporator 261 is compressed by the condenser 262 in the tank 270, and heats the disinfectant solution V in the tank 270.

Thereafter, the compressed air HA is converted into the cooled air CA by the expansion valve 263, and thereafter, is used for bringing the liquid droplets of the gas-liquid two-phase flow M into a state of ice in the evaporator 261. By such a configuration, the same effect as the abovementioned Fig. 12 also can be obtained, and in addition, the disinfectant solution in the tank 270 can be heated more effectively than Fig. 12 since the condenser 262 is provided in the tank 270.

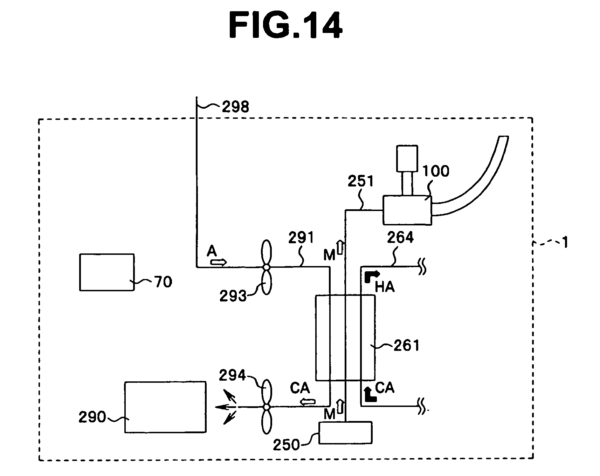

Hereinafter, still another modified example will be shown with use of Fig. 14. Fig. 14 is a diagram showing a configuration of a modified example which cools the air which cools an electric substrate in a washing and disinfecting apparatus, in each of the evaporators in Figs. 12 and 13.

Various electronic components 290 provided in the washing and disinfecting apparatus 1 generate heat with drive. The electronic components 290 sometimes fail when they generate heat excessively. Therefore, generally in the washing and disinfecting apparatus 1, a fan is provided in the apparatus 1, and a hole which communicates with an outside of the apparatus 1 is provided, so that the air introduced into the apparatus 1 from the outside of the apparatus 1 by drive of the fan is supplied to the electronic components 290, and thereby, the electronic components 290 is cooled by the air.

The air which is supplied to the electronic components 290 may be cooled by using the evaporator 261 which converts the liquid droplets in the gas-liquid two-phase flow M shown in the abovementioned Figs. 12 and 13 into ice grains I.

Specifically, as shown in Fig. 14, the evaporator 261 is provided in an intermediate position of a cooling air supply channel 291 with one end being located outside the washing and disinfecting apparatus 1 as an introduction port 298 and the other end being located to be opposed to the electronic components 290.

Further, a fan 293 for introducing the air A into the apparatus 1 from the outside of the washing and disinfecting apparatus 1 is provided between the introduction port 298 of the cooling air supply channel 291 and the evaporator 261, and a fan 294 for supplying the air CA which is cooled by the evaporator 261 to the electronic components 290 is provided between the evaporator 261 and the electronic component 290. The fans 293 and 294 are driven by drive control of the control unit 70.

According to such a configuration, the air CA cooled by the evaporator 261 can be reliably supplied to the electronic components 290, and therefore, the electronic components 290 can be more reliably cooled than conventionally cooled. The other effects are the same as those in Figs. 12 and 13.

## Claims

1. An endoscope washing and disinfecting apparatus which (1) is adapted to automatically wash and disinfect an endoscope (100), the apparatus (1) comprising:
- a gas supply section (44, 45) which is adapted to supply a gas to an endoscope channel (100k) of the endoscope (100);
- a liquid supply section (5, 112) which is adapted to supply a liquid to the endoscope channel (100k);
- a fluid supply channel (21; 221; 251) which is connectable to a mouthpiece of the endoscope channel (100k), and which further is adapted to supply at least one of the gas from the gas supply section and the liquid from the liquid supply section to the endoscope channel (100k); and
- a liquid droplet mixing section (110, 115) which is adapted to convert the liquid supplied from the liquid supply section into liquid droplets (120), and to mix the liquid droplets (120) into the gas flowing through the fluid supply channel (21) with a size of the liquid droplets being changeable, to thereby supply a gas-liquid two phase flow (M), in which the liquid droplets are mixed at a set ratio into the gas, into the endoscope channel (100k) through the fluid supply channel (21; 221; 251),
**characterized in that** the liquid droplet mixing section further is adapted to control a change of a size of the liquid droplets, and to mix liquid droplets, the size of which has been changed, into the gas flowing through the fluid supply channel (21).

2. The endoscope washing and disinfecting apparatus according to claim 1,
wherein the liquid droplet mixing section is adapted to control the change of the size of the liquid droplets (120) by regulating a liquid supply pressure of the liquid, which is supplied from the liquid supply section to the liquid droplet mixing section.

3. The endoscope washing and disinfecting apparatus according to claim 1 or 2,
wherein the liquid droplet mixing section is configured by a piezoelectric element injector (110) which is adapted to convert the liquid supplied from the liquid supply section into liquid droplets (120), and which is adapted to control a change of the size of the liquid droplets (120) by changing a supply voltage.

4. The endoscope washing and disinfecting apparatus according to claim 1,
wherein the liquid droplet mixing section comprises an ultrasonic generating section (200) which is adapted to convert the liquid supplied from the liquid supply section into liquid droplets by ultrasound, and which is adapted to control a change of the size of the liquid droplets by changing an ultrasonic frequency.

5. The endoscope washing and disinfecting apparatus according to claim 1, further comprising:
a heat exchanger (150) which is adapted to cool the gas supplied from the gas supply section,
wherein the liquid droplet mixing section is adapted to mix the liquid droplets (120) into the gas cooled by the heat exchanger, to thereby bring the liquid droplets (120) mixed into the gas into a state of ice.

6. The endoscope washing and disinfecting apparatus according to claim 1, further comprising:
a heat pump evaporator (261) which is adapted to bring the liquid droplets (120) mixed in the gas-liquid two-phase flow (M) into a state of ice; and
a heat pump condenser (262) which is adapted to heat a liquid to be used for washing and disinfecting the endoscope (100) by a gas heated by the heat pump evaporator (261).

7. A method of washing a endoscopy (100) using an endoscope washing and disinfecting apparatus (1) as defined in claim 1 which automatically washes and disinfects the endoscope (100), the method comprising:
- connecting a mouthpiece of an endoscope channel (100k) of the endoscope (100) to a fluid supply channel (21; 221; 251) of the endoscope washing and disinfecting apparatus (1),
- converting the liquid supplied from the liquid supply section into liquid droplets (120) and mixing the liquid droplets (120) into the gas which is supplied from the gas supply section and flows through the fluid supply channel (21; 221; 251) by means of a liquid droplet mixing section provided in an intermediate position of the fluid supply channel (21; 221; 251), with the size of the liquid droplets being changeable, to thereby supply a gas-liquid two-phase flow (M), in which the liquid droplets (120) are mixed at a set ratio into the gas, into the endoscope channel (100k) via the fluid supply channel (21; 221; 251),
**characterized in that** the liquid droplet mixing section controls a change of a size of the liquid droplets (120) to mix liquid droplets, the size of which has been changed, into the fluid supply channel (21; 221; 251).

8. The method according to claim 7,
wherein the liquid droplet mixing section controls the change of a size of the liquid droplets (120) by regulating a liquid supply pressure of the liquid, which is supplied from the liquid supply section to the liquid droplet mixing section.

9. The method according to claim 7,
wherein, after supplying a set amount of the gas-liquid two-phase flow (M) to the endoscope channel (100k), in which liquid droplets (120) of a first size are mixed into the gas, a set amount of the gas-liquid two-phase flow (M) is supplied to the endoscope channel (100k), in which liquid droplets (120) of a second size smaller than the first size are mixed into the gas.

10. The method according to claim 7,
wherein the gas supplied from the gas supply section is cooled by a heat exchanger (150) and the liquid droplets (120), by the liquid droplet mixing section, are mixed into the gas cooled by the heat exchanger (150) to thereby bring the liquid droplets (120) mixed into the gas into a state of ice.

## Patentansprüche

1. Reinigungs- und Desinfektionsgerät für Endoskope, das (1) ein Endoskop (100) automatisch zu reinigen und zu desinfizieren vermag, wobei das Gerät (1) umfasst:
- einen Gaszuführbereich (44, 45), der einem Endoskopkanal (100k) des Endoskops (100) ein Gas zuzuführen vermag;
- einen Flüssigkeitszuführbereich (5, 112), der dem Endoskopkanal (100k) eine Flüssigkeit zuzuführen vermag;
- einen Fluidzuführkanal (21; 221; 251), der mit einem Mundstück des Endoskopkanals (100k) verbindbar ist, und der ferner dem Endoskopkanal (100k) das Gas vom Gaszuführbereich und/oder die Flüssigkeit vom Flüssigkeitszuführbereich zuzuführen vermag; und
- einen Flüssigkeitströpfchen-Mischbereich (110, 115), der die vom Flüssigkeitszuführbereich zugeführte Flüssigkeit in Flüssigkeitströpfchen (120) umzuformen und die Flüssigkeitströpfchen (120) dem durch den Fluidzuführkanal (21) strömenden Gas mit einer änderbaren Größe der Flüssigkeitströpfchen beizumischen vermag, um dadurch einen zweiphasigen Gas-Flüssigkeits-Strom (M), in welchem die Flüssigkeitströpfchen in einem gegebenen Verhältnis dem Gas beigemischt sind, durch den Fluidzuführkanal (21; 221; 251) in den Endoskopkanal (100k) zuzuführen,
**dadurch gekennzeichnet, dass** der Flüssigkeitströpfchen-Mischbereich ferner eine Änderung der Größe der Flüssigkeitströpfchen zu steuern und Flüssigkeitströpfchen, deren Größe verändert worden ist, dem durch den Fluidzuführkanal (21) strömenden Gas beizumischen vermag.

2. Reinigungs- und Desinfektionsgerät für Endoskope nach Anspruch 1,
wobei der Flüssigkeitströpfchen-Mischbereich die Änderung der Größe der Flüssigkeitströpfchen (120) zu steuern vermag, indem ein Flüssigkeitszuführdruck der Flüssigkeit, die vom Flüssigkeitszuführbereich zum Flüssigkeitströpfchen-Mischbereich zugeführt wird, eingestellt wird.

3. Reinigungs- und Desinfektionsgerät für Endoskope nach Anspruch 1 oder 2,
wobei der Flüssigkeitströpfchen-Mischbereich durch einen Piezoelement-Injektor (110) gebildet ist, welche die vom Flüssigkeitszuführbereich zugeführte Flüssigkeit in Flüssigkeitströpfchen (120) umzuformen vermag und welche eine Änderung der Größe der Flüssigkeitströpfchen (120) durch Ändern einer Versorgungsspannung zu steuern vermag.

4. Reinigungs- und Desinfektionsgerät für Endoskope nach Anspruch 1,
wobei der Flüssigkeitströpfchen-Mischbereich einen Ultraschallerzeugungsbereich (200) umfasst, welcher die vom Flüssigkeitszuführbereich zugeführte Flüssigkeit durch Ultraschall in Flüssigkeitströpfchen umzuformen vermag und welcher eine Änderung der Größe der Flüssigkeitströpfchen durch Ändern einer Ultraschallfrequenz zu steuern vermag.

5. Reinigungs- und Desinfektionsgerät für Endoskope nach Anspruch 1, das ferner umfasst:
einen Wärmetauscher (150), der das vom Gaszuführbereich zugeführte Gas zu kühlen vermag,
wobei der Flüssigkeitströpfchen-Mischbereich die Flüssigkeitströpfchen (120) dem durch den Wärmetauscher gekühlten Gas beizumischen vermag, um dadurch die dem Gas beigemischten Flüssigkeitströpfchen (120) in einen gefrorenen Zustand zu versetzen.

6. Reinigungs- und Desinfektionsgerät für Endoskope nach Anspruch 1, das ferner umfasst:
einen Wärmepumpenverdampfer (261), welcher die dem zweiphasigen Gas-Flüssigkeits-Strom (M) beigemischten Flüssigkeitströpfchen (120) in einen gefrorenen Zustand zu bringen vermag; und
einen Wärmepumpenkondensator (262), welcher eine Flüssigkeit, die zum Reinigen und Desinfizieren des Endoskops (100) benutzt werden soll, durch ein vom Wärmepumpenverdampfer (261) erwärmtes Gas zu erwärmen vermag.

7. Verfahren zum Reinigen eines Endoskops (100) unter Verwendung eines Reinigungs- und Desinfektionsgeräts für Endoskope (1) nach Anspruch 1, welches das Endoskop (100) automatisch reinigt und desinfiziert, wobei das Verfahren umfasst:
- Verbinden eines Mundstücks eines Endoskopkanals (100k) des Endoskops (100) mit einem Fluidzuführkanal (21; 221; 251) des Reinigungs- und Desinfektionsgeräts (1) für Endoskope,
- Umformen der vom Flüssigkeitszuführbereich zugeführten Flüssigkeit zu Flüssigkeitströpfchen (120) und Beimischen der Flüssigkeitströpfchen (120) zu dem Gas, das vom Gaszuführbereich zugeführt wird und durch den Fluidzuführkanal (21; 221; 251) strömt, mittels eines Flüssigkeitströpfchen-Mischbereichs, der an einer Zwischenposition des Fluidzuführkanals (21; 221; 251) vorgesehen ist, wobei die Größe der Flüssigkeitströpfchen änderbar ist, um dem Endoskopkanal (100k) dadurch einen zweiphasigen Gas-Flüssigkeits-Strom (M), bei dem die Flüssigkeitströpfchen (120) in einem gegebenen Verhältnis dem Gas beigemischt werden, über den Fluidzuführkanal (21; 221; 251) zuzuführen,
**dadurch gekennzeichnet, dass** der Flüssigkeitströpfchen-Mischbereich eine Änderung der Größe der Flüssigkeitströpfchen (120) steuert, um Flüssigkeitströpfchen, deren Größe geändert worden ist, dem Fluidzuführkanal (21; 221; 251) beizumischen.

8. Verfahren nach Anspruch 7,
wobei der Flüssigkeitströpfchen-Mischbereich die Änderung der Größe der Flüssigkeitströpfchen (120) steuert, indem ein Flüssigkeitszuführdruck der Flüssigkeit, die vom Flüssigkeitszuführbereich zum Flüssigkeitströpfchen-Mischbereich zugeführt wird, eingestellt wird.

9. Verfahren nach Anspruch 7,
wobei nach dem Zuführen einer bestimmten Menge des zweiphasigen Gas-Flüssigkeits-Stroms (M) zum Endoskopkanal (100k), in welchem Flüssigkeitströpfchen (120) einer ersten Größe dem Gas beigemischt werden, eine bestimmte Menge des zweiphasigen Gas-Flüssigkeits-Stroms (M) dem Endoskopkanal (100k) zugeführt wird, in welchem Flüssigkeitströpfchen (120) einer zweiten Größe, die geringer als die erste Größe ist, dem Gas beigemischt werden.

10. Verfahren nach Anspruch 7,
wobei das von Gaszuführbereich zugeführte Gas durch einen Wärmetauscher (150) gekühlt wird und die Flüssigkeitströpfchen (120) durch den Flüssigkeitströpfchen-Mischbereich dem Gas beigemischt werden, welches durch den Wärmetauscher (150) gekühlt wird, um dadurch die dem Gas beigemischten Flüssigkeitströpfchen (120) in einen gefrorenen Zustand zu versetzen.

## Revendications

1. Appareil de nettoyage et de désinfection d'un endoscope (1) qui est adapté pour nettoyer et désinfecter automatiquement un endoscope (100), l'appareil (1) comprenant :
- une section d'alimentation en gaz (44, 45) qui est adaptée pour délivrer un gaz vers un canal d'endoscope (100k) de l'endoscope (100) ;
- une section d'alimentation en liquide (5, 112) qui est adaptée pour délivrer un liquide vers le canal d'endoscope (100k) ;
- un canal d'alimentation en fluide (21 ; 221 ; 251) qui peut être connecté à une embouchure du canal d'endoscope (100k), et qui est en outre adapté pour délivrer au moins l'un parmi le gaz provenant de la section d'alimentation en gaz et le liquide provenant de la section d'alimentation en liquide vers le canal d'endoscope (100k) ; et
- une section de mélange de gouttelettes liquides (110, 115) qui est adaptée pour convertir le liquide délivré depuis la section d'alimentation en liquide en gouttelettes liquides (120), et pour mélanger les gouttelettes liquides (120) dans le gaz circulant à travers le canal d'alimentation en fluide (21) avec une taille de gouttelettes liquides pouvant changer, pour de ce fait délivrer un écoulement diphasique gaz-liquide (M) dans lequel les gouttelettes liquides sont mélangées à un rapport établi dans le gaz, dans le canal d'endoscope (100k) à travers le canal d'alimentation en fluide (21 ; 221 ; 251),
**caractérisé en ce que** la section de mélange de gouttelettes liquides est en outre adaptée pour commander un changement d'une taille des gouttelettes liquides, et pour mélanger les gouttelettes liquides, dont la taille a été changée, dans le gaz circulant à travers le canal d'alimentation en fluide (21).

2. Appareil de nettoyage et de désinfection d'un endoscope selon la revendication 1,
dans lequel la section de mélange de gouttelettes liquides est adaptée pour commander le changement de la taille des gouttelettes liquides (120) en régulant une pression d'alimentation en liquide du liquide, qui est délivré depuis la section d'alimentation en liquide vers la section de mélange de gouttelettes liquides.

3. Appareil de nettoyage et de désinfection d'un endoscope selon la revendication 1 ou 2,
dans lequel la section de mélange de gouttelettes liquides est configurée par un injecteur à élément piézo-électrique (110) qui est adapté pour convertir le liquide délivré depuis la section d'alimentation en liquide en gouttelettes liquides (120), et qui est adapté pour commander un changement de la taille des gouttelettes liquides (120) en changeant une tension d'alimentation.

4. Appareil de nettoyage et de désinfection d'un endoscope selon la revendication 1,
dans lequel la section de mélange de gouttelettes liquides comprend une section de génération ultrasonore (200) qui est adaptée pour convertir le liquide délivré depuis la section d'alimentation en liquide en gouttelettes liquides par ultrasons, et qui est adaptée pour commander un changement de la taille des gouttelettes liquides en changeant une fréquence ultrasonore.

5. Appareil de nettoyage et de désinfection d'un endoscope selon la revendication 1, comprenant en outre :
un échangeur de chaleur (150) qui est adapté pour refroidir le gaz délivré depuis la section d'alimentation en gaz,
dans lequel la section de mélange de gouttelettes liquides est adaptée pour mélanger les gouttelettes liquides (120) dans le gaz refroidi par l'échangeur de chaleur, pour de ce fait amener les gouttelettes liquides (120) mélangées dans le gaz dans un état de glace.

6. Appareil de nettoyage et de désinfection d'un endoscope selon la revendication 1, comprenant en outre :
un évaporateur de pompe à chaleur (261) qui est adapté pour amener les gouttelettes liquides (120) mélangées dans l'écoulement diphasique gaz-liquide (M) dans un état de glace ; et
un condenseur de pompe à chaleur (262) qui est adapté pour chauffer un liquide devant être utilisé pour nettoyer et désinfecter l'endoscope (100) par un gaz chauffé par l'évaporateur de pompe à chaleur (261).

7. Procédé de nettoyage d'un endoscope (100) utilisant un appareil de nettoyage et de désinfection d'endoscope (1) selon la revendication 1, qui nettoie et désinfecte automatiquement l'endoscope (100), le procédé comprenant les étapes consistant à :
- raccorder une embouchure d'un canal d'endoscope (100k) de l'endoscope (100) à un canal d'alimentation en fluide (21 ; 221 ; 251) de l'appareil de nettoyage et de désinfection d'endoscope (1),
- convertir le liquide délivré depuis la section d'alimentation en liquide en gouttelettes liquides (120) et mélanger les gouttelettes liquides (120) dans le gaz qui est délivré depuis la section d'alimentation en gaz et circule à travers le canal d'alimentation en fluide (21 ; 221 ; 251) au moyen d'une section de mélange de gouttelettes liquides prévue dans une position intermédiaire du canal d'alimentation en fluide (21 ; 221 ; 251), avec la taille des gouttelettes liquides pouvant être changée, pour de ce fait délivrer un écoulement diphasique gaz-liquide (M), dans lequel les gouttelettes liquides (120) sont mélangées à un rapport établi dans le gaz, dans le canal d'endoscope (100k) via le canal d'alimentation en fluide (21 ; 221 ; 251),
**caractérisé en ce que** la section de mélange de gouttelettes liquides commande le changement d'une taille des gouttelettes liquides (120) pour mélanger les gouttelettes liquides, dont la taille a été changée, dans le canal d'alimentation en fluide (21 ; 221 ; 251).

8. Procédé selon la revendication 7,
dans lequel la section de mélange de gouttelettes liquides commande le changement d'une taille des gouttelettes liquides (120) en régulant une pression d'alimentation en liquide du liquide, qui est délivré depuis la section d'alimentation en liquide vers la section de mélange de gouttelettes liquides.

9. Procédé selon la revendication 7,
dans lequel, après avoir délivré une quantité établie d'écoulement diphasique gaz-liquide (M) vers le canal d'endoscope (100k), dans lequel des gouttelettes liquides (120) d'une première taille sont mélangées dans le gaz, une quantité établie de l'écoulement diphasique gaz-liquide (M) est délivrée dans le canal d'endoscope (100k), dans lequel des gouttelettes liquides (120) d'une deuxième taille inférieure à la première taille sont mélangées dans le gaz.

10. Procédé selon la revendication 7,
dans lequel le gaz délivré depuis la section d'alimentation en gaz est refroidi par un échangeur de chaleur (150) et les gouttelettes liquides (120), par la section de mélange de gouttelettes liquides, sont mélangées dans le gaz refroidi par l'échangeur de chaleur (150) pour de ce fait amener les gouttelettes liquides (120) mélangées dans le gaz dans un état de glace.
